# EUROPEAN PATENT APPLICATION

(11) **EP 2 325 864 A1**
(43) Date of publication of application: **25.05.2011**
(21) Application number: 10191809.2
(22) Date of filing: 19.11.2010
(51) Int. Cl.: H01J 49/16

(54) **High throughput apparatus and method for multiple sample analysis**

(30) Priority: 19.11.2009 KR 20090112077
(71) Applicant: Korea Basic Science Institute, Daejeon 305-333 (KR)
(72) Inventor: Choi, Myoung Choul, 142-771, Seoul (KR); Kim, Hyun Sik, 305-350, Daejeon (KR); Yoo, Jong Shin, 305-308, Daejeon (KR); Kim, Geon Hee, 305-749, Daejeon (KR); Chang, Ki Soo, 306-761, Daejeon (KR)
(74) Representative: Ferreccio, Rinaldo

(57) **Abstract**

A high throughput apparatus for multiple sample analysis is disclosed. The high through apparatus for multiple sample analysis may include: a laser light source; a lens array configured to focus laser irradiated by the laser light source into a plurality of focused laser beams; and a focusing unit disposed between the lens array and a sample, the focusing unit configured to focus ions produced from the sample by the plurality of focused laser beams into a plurality of ion beams. A high throughput method for multiple sample analysis may include: producing a plurality of focused laser beams by focusing laser; ionizing a sample by irradiating the plurality of focused laser beams to the sample; and producing a plurality of ion beams by focusing ions, wherein the ions are produced from the sample by the plurality of focused laser beams.

## Description

### BACKGROUND

### 1. Field

Embodiments relate to high throughput apparatus and method for multiple sample analysis.

### 2. Description of the Related Art

Matrix-assisted laser desorption/ionization (MALDI) is a technique used in mass spectrometry whereby a sample is ionized by means of laser and the mass of the ionized sample is analyzed. The MALDI mass spectrometry may be employed for analysis of biomolecules including proteins, peptides and sugars. Further, it may be used for the analysis of organic molecules including polymers, dendrimers and other macromolecules.

For MALDI mass spectrometry, the sample may be mixed with a matrix for facilitating ionization and then laser may be irradiated to the sample. When the sample is ionized by the laser, the ionized analytes may be transferred to a mass spectrometer. By analyzing the ions using the mass spectrometer, the mass of the analytes may be determined. Because the ionization occurs where the laser is applied to the sample, the aforesaid procedure has to be repeatedly performed for the whole sample to find out the overall mass distribution of the sample.

### SUM0MARY

Embodiments are directed to providing high throughput apparatus and method for multiple sample analysis configured to produce an array of a plurality of focused laser beams by focusing laser and to irradiate them to a sample so as to ionize a sample with a large area or a large number of samples at the same time.

According to an embodiment, a high throughput apparatus for multiple sample analysis may include: a laser light source; a lens array configured to focus laser irradiated by the laser light source into a plurality of focused laser beams; and a focusing unit disposed between the lens array and a sample, the focusing unit configured to focus ions produced from the sample by the plurality of focused laser beams into a plurality of ion beams.

According to another embodiment, a high throughput method for multiple sample analysis may include: producing a plurality of focused laser beams by focusing laser; ionizing a sample by irradiating the plurality of focused laser beams to the sample; and producing a plurality of ion beams by focusing ions, wherein the ions are produced from the sample by the plurality of focused laser beams.

### BRIEF DESCRIPTION OF THE DRAWINGS

The above and other aspects, features and advantages of the disclosed exemplary embodiments will be more apparent from the following detailed description taken in conjunction with the accompanying drawings in which:
FIG. 1 is a schematic view of a high throughput apparatus for multiple sample analysis according to an embodiment;
FIG. 2 is a schematic perspective view of a lens array included in a high throughput apparatus for multiple sample analysis according to an embodiment;
FIG. 3 is a cross-sectional view of a focusing unit included in a high throughput apparatus for multiple sample analysis according to an embodiment;
FIG. 4 is a front view of the focusing unit illustrated in FIG. 3;
FIG. 5 is a cross-sectional view of a focusing unit included in a high throughput apparatus for multiple sample analysis according to another embodiment;
FIG. 6 is a cross-sectional view of an ion trap included in a high throughput apparatus for multiple sample analysis according to an embodiment;
FIG. 7 is a schematic exploded perspective view of the ion trap illustrated in FIG. 6; and
FIG. 8 is a flow chart of a high throughput method for multiple sample analysis according to an embodiment.

### DETAILED DESCRIPTION

Exemplary embodiments now will be described more fully hereinafter with reference to the accompanying drawings, in which exemplary embodiments are shown. The present disclosure may, however, be embodied in many different forms and should not be construed as limited to the exemplary embodiments set forth therein. Rather, these exemplary embodiments are provided so that the present disclosure will be thorough and complete, and will fully convey the scope of the present disclosure to those skilled in the art. In the description, details of well-known features and techniques may be omitted to avoid unnecessarily obscuring the presented embodiments.

The terminology used herein is for the purpose of describing particular embodiments only and is not intended to be limiting of the present disclosure. As used herein, the singular forms "a", "an" and "the" are intended to include the plural forms as well, unless the context clearly indicates otherwise. Furthermore, the use of the terms a, an, etc. does not denote a limitation of quantity, but rather denotes the presence of at least one of the referenced item. The use of the terms "first", "second", and the like does not imply any particular order, but they are included to identify individual elements. Moreover, the use of the terms first, second, etc. does not denote any order or importance, but rather the terms first, second, etc. are used to distinguish one element from another. It will be further understood that the terms "comprises" and/or "comprising", or "includes" and/or "including" when used in this specification, specify the presence of stated features, regions, integers, steps, operations, elements, and/or components, but do not preclude the presence or addition of one or more other features, regions, integers, steps, operations, elements, components, and/or groups thereof.

Unless otherwise defined, all terms (including technical and scientific terms) used herein have the same meaning as commonly understood by one of ordinary skill in the art. It will be further understood that terms, such as those defined in commonly used dictionaries, should be interpreted as having a meaning that is consistent with their meaning in the context of the relevant art and the present disclosure, and will not be interpreted in an idealized or overly formal sense unless expressly so defined herein.

In the drawings, like reference numerals in the drawings denote like elements. The shape, size and regions, and the like, of the drawing may be exaggerated for clarity.

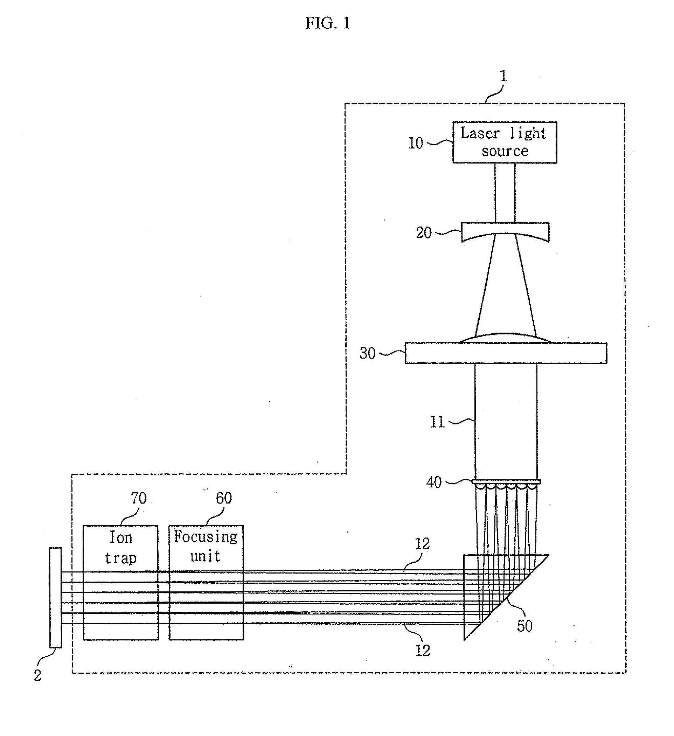

FIG. 1 is a schematic view of a high throughput apparatus for multiple sample analysis according to an embodiment.

Referring to FIG. 1, a high throughput apparatus 1 for multiple sample analysis may comprise a laser light source 10, a lens array 40 and a focusing unit 60. By the high throughput apparatus 1 for multiple sample analysis, a plurality of focused laser beams 12 may be irradiated to a sample 2 in an array pattern. When the laser beams 12 are irradiated, the sample 2 may be ionized and ions may be produced therefrom. Then, the high throughput apparatus 1 for multiple sample analysis may focus the ions into an array of a plurality of ion beams (not shown).

As used herein, an array refers to an arrangement of a plurality of elements in one or more row(s) and/or one or more column(s). The array includes an arrangement of a plurality of elements in a single row or in a single column. Further, in an array consisting of a plurality of rows and/or a plurality of columns, the number of elements in each row and/or column may not be necessarily the same.

The laser light source 10 is a device for irradiating laser 11, which is an energy source for ionizing the sample 2. The laser light source 10 is not limited to a device or setup for irradiation of a specific type of laser, but may be any device irradiating laser of arbitrary wavelength. For example, the laser light source 10 may be configured to output laser of appropriate wavelength and/or intensity depending on the sample 2, the matrix included in the sample 2, or the like.

The laser 11 irradiated by the laser light source 10 may be propagated toward the lens array 40. In an embodiment, the high throughput apparatus 1 for multiple sample analysis may further comprise a dispersing lens 20 and/or an intermediate control lens 30 disposed between the laser light source 10 and the lens array 40. The dispersing lens 20 and/or the intermediate control lens 30 may be made of a substantially transparent material such as glass.

The dispersing lens 20 may disperse the laser 11 so as to increase the area of the region at which the laser 11 irradiated by the laser light source 10 arrives. For example, the dispersing lens 20 may disperse the laser 11 so that the laser 11 may be incident on substantially the whole area of the lens array 40. The dispersing lens 20 may be a concave lens, an aspheric lens or other adequate optical element.

The intermediate control lens 30 may at least partially focus the dispersed laser 11 and transmit it to the lens array 40. For example, the intermediate control lens 30 may focus the laser 11 such that the laser 11 may be incident on the surface of the lens array 40 substantially vertically. The intermediate control lens 30 may be a convex lens, an aspheric lens or other adequate optical element.

Depending on the wavelength or intensity of the laser 11 irradiated by the laser light source 10, the distance between the laser light source 10 and the lens array 40, the arrangement thereof, or the like, the dispersing lens 20 and/or the intermediate control lens 30 may be unnecessary. That is to say, the laser 11 may be directly irradiated from the laser light source 10 to the lens array 40 without an additional optical element.

The lens array 40 may comprise a plurality of lenses arranged in an array pattern. That is to say, the lens array 40 may be an aspheric lens with a plurality of lenses arranged on a plane, instead of a lens having a convex or concave spherical surface. When the laser 11 is irradiated on the lens array 40, each of the lenses may focus the laser 11 to produce focused laser beam 12. Accordingly, a plurality of focused laser beams 12 arranged in an array pattern may be output from the lens array 40.

In an embodiment, a prism 50 may be provided between the lens array 40 and the sample 2. The prism 50 reflects the plurality of focused laser beams 12 produced by the lens array 40, thereby steering the focused laser beams 12 toward the sample 2. However, the prism 50 may be unnecessary depending on the arrangement of the sample 2, the laser light source 10 and/or the lens array 40.

The plurality of focused laser beams 12 focused by the lens array 40 may be irradiated to the sample 2. The plurality of focused laser beams 12 may be irradiated to the sample 2 after passing through the focusing unit 60 and/or an ion trap 70. For this purpose, the focusing unit 60 and/or the ion trap 70 may include at least in part a laser-transmittable zone.

As used herein, the sample is intended to refer to a material that can be ionized by laser, including a matrix for facilitating the ionization. For example, the sample 2 may biomolecules such as proteins, peptides and sugars, organic molecules including polymers, dendrimers and other macromolecules, inorganic molecules, or other suitable materials including a matrix.

When a laser of a specific wavelength is irradiated to the sample 2, the matrix included in the sample 2 may absorb laser energy and facilitate the ionization of the sample 2. For this purpose, the matrix may be a material having a crystallized molecular structure. For example, 3,5-dimethoxy-4-hydroxycinnamic acid, α-cyano-4-hydroxycinnamic acid, 2,5-dihydroxybenzoic acid, or other suitable material may be used as the matrix.

When the focused laser beams 12 are irradiated to the sample 2, the sample 2 may be ionized at the region where the focused laser beams 12 are irradiated and ions may be produced as a result of the ionization. Since the focused laser beams 12 are arranged in an array pattern, the ions may be simultaneously produced over a relatively large area of the sample 2. Also, depending on the size of the sample 2, a plurality of samples 2 may be ionized at the same time.

The focusing unit 60 may focus the ions produced as the sample 2 is ionized into a plurality of ion beams (not shown). For example, the focusing unit 60 may focus a plurality of ion beams in an array pattern which is the same as the array of the plurality of focused laser beams 12. The focusing unit 60 may comprise, for example, an einzel lens as a device to focus the ions using an electromagnetic field.

In an embodiment, the high throughput apparatus 1 for multiple sample analysis may comprise an ion trap 70 between the sample 2 and the focusing unit 60. The ion trap 70 may be a device for trapping the ions produced as the sample 2 is ionized by the laser. Since charged particles such as ions are affected by an electric field, ions may be trapped in the ion trap 70 by applying an adequate trapping field. For example, the ion trap 70 may be a Paul trap or other adequate devices.

Immediately after being ionized by the laser, the ions have very high temperatures. This may be unfavorable in treating the ions in the following processes such as mass spectrometry. If the ions can be trapped in the ion trap 70 using an electromagnetic field, the trapped ions may be allowed to cool.

In an embodiment, in response to a signal applied to the ion trap 70, the trapped ions may be selectively emitted and transmitted to the focusing unit 60. For example, a radio frequency (RF) signal may be applied to the ion trap 70 from an external power source (not shown). The ion trap 70 may be configured such that ions with specific mass corresponding to the applied RF signal are selectively emitted among the trapped ions. In this case, since specific ions may be output excluding impurities, mass spectrometry or the like on the sample 2 may be carried out conveniently.

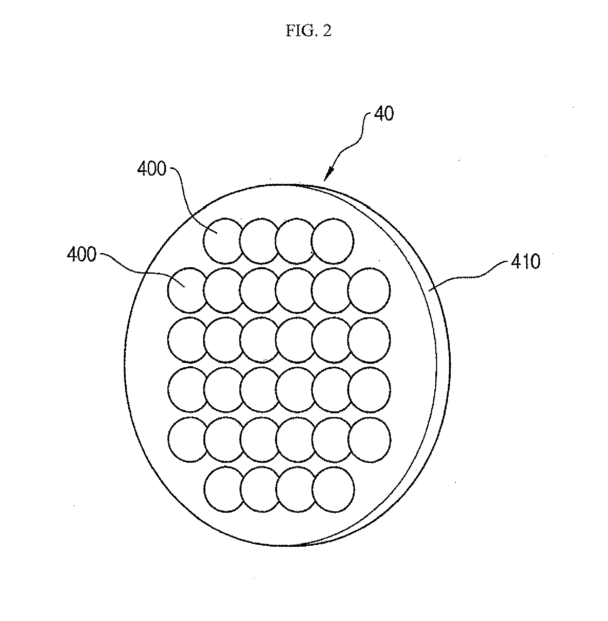

FIG. 2 is a schematic perspective view of a lens array included in a high throughput apparatus for multiple sample analysis according to an embodiment.

Referring to FIG. 2, the lens array 40 may comprise a plurality of lenses 400 arranged on a plane in an array pattern. For example, the plurality of lenses 400 may be arranged on a plate-shaped platform 410 in an array pattern. Each of the lenses 400 may focus the laser irradiated to the lens 400 and produce a focused laser beam. Since the plurality of lenses 400 are arranged in an array pattern, a plurality of focused laser beams arranged in an array pattern may be output from the lens array 40.

In an embodiment, each of the lenses 400 may have a micrometer (µm) scale diameter. Also, the number of the lenses 400 illustrated in FIG. 2 is only exemplary. For example, the plurality of lenses 400 may be arranged in a square array pattern of about 200 rows and 200 columns. The size, number and arrangement of the plurality of lenses 400 may be determined appropriately depending on the size and shape of the region to which the laser beams are to be irradiated.

The platform 410 and/or the lenses 400 may be made of a laser-transmittable material and may be made of a substantially transparent material. For example, the platform 410 and/or lenses 400 may be made of glass, zinc selenide (ZnSe) or other suitable material. Each of the lenses 400 may be processed into a desired shape, for example, by cutting with a diamond tool. The shape of the lens 400 may be adequately determined based on the focal length or the like of the laser beams to be outputted.

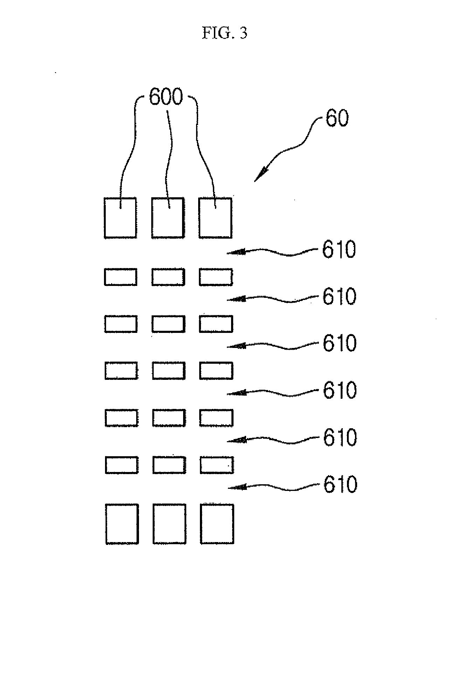

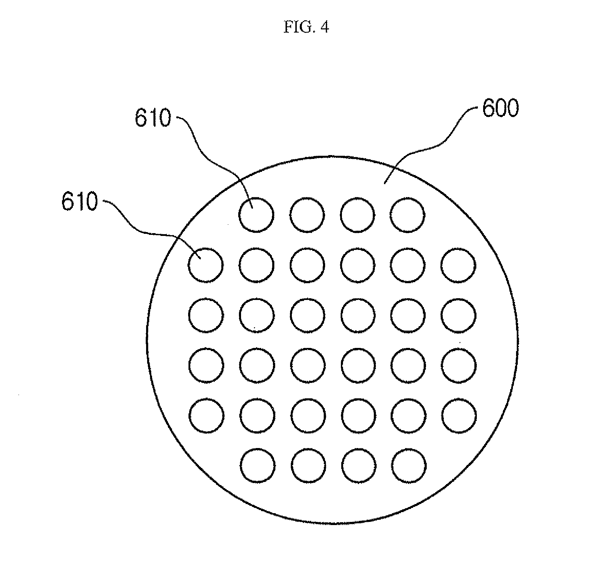

FIG. 3 is a cross-sectional view of the focusing unit 60 included in a high throughput apparatus for multiple sample analysis according to an embodiment, and FIG. 4 is a front view of the focusing unit 60 illustrated in FIG. 3. FIG. 3 shows a cross-sectional view of the focusing unit 60 along a direction perpendicular to the path of the laser beams and ions.

The focusing unit 60 illustrated in FIG. 3 and FIG. 4 is one using an einzel lens for focusing the ions. Referring to FIGS. 3 and 4, the focusing unit 60 may comprise a plurality of plates 600 spaced apart from each other and arranged to face each other along a direction. Each of the plates 600 may be made of metal or other suitable conducting material. The diameter and thickness of each plate 600, the spacing between the plates 600, or the like may be determined adequately depending on the characteristics of the ion beams to be focused.

A plurality of holes 610 may be formed on each of the plates 600. The plurality of holes 610 may be arranged in an array pattern. For example, the plurality of holes 610 may be arranged in an array pattern which is the same as the arrangement of the plurality of lens of the lens array. That is to say, the holes 610 may be disposed in positions corresponding to the positions of the lenses of the lens array. Further, each hole 610 may have a micrometer (µm) scale diameter.

When the plurality of focused laser beams are propagated by the lens array, each of the focused laser beams may pass through the hole 610 and then be irradiated to the sample. Then, when ions are produced as the sample is ionized by the irradiated laser beams, the produced ions pass through the holes 610.

An electric power may be applied between the plurality of plates 600. By controlling the applied power, the ions passing through the holes 610 may be focused into ion beams. For example, among the plurality of plates 600, a relatively larger voltage may be applied to the plate 600 located in the middle as compared to the other plates 600. A description of the operation of the einzel lens will be omitted since it is well known to those skilled in the art.

Although an example using the einzel lens for the focusing unit 60 was described above, the configuration of the focusing unit 60 is not limited thereto. For example, the number and shape of the plates 600, the shape and arrangement of the holes 610, or the like may be different from those illustrated in the figures. In addition, the focusing unit 60 may be configured otherwise to appropriately focus the ions into ion beams.

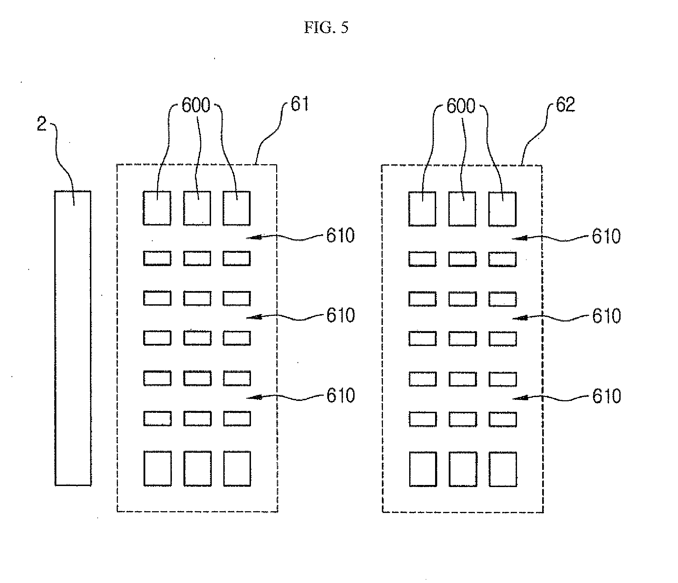

FIG. 5 is a cross-sectional view of a focusing unit included in a high throughput apparatus for multiple sample analysis according to another embodiment.

Referring to FIG. 5, a high throughput apparatus for multiple sample analysis may comprise a plurality of focusing units 61, 62. As described above referring to FIGS. 3 and 4, each of the focusing units 61, 62 may comprise a plurality of plates 600 and a plurality of holes 610 formed on each plate 600 and arranged in an array pattern. A detailed description on the configuration of the high throughput apparatus for multiple sample analysis excluding the focusing units 61, 62 will be omitted since it is the same as that of the embodiment describe above with respect to FIG. 1.

In the high throughput apparatus for multiple sample analysis, each of the focusing units 61, 62 may be used for different purposes by controlling the power applied to each of the focusing units 61, 62. For example, a first focusing unit 61 relatively closer to a sample 2 may be used to accelerate ions, and a second focusing unit 62 which is relatively distant from the sample 2 may be used to focus the accelerated ions.

To use the focusing units 61, 62 for different purposes as such, the power applied to the plurality of plates 600 constituting each of the focusing units 61, 62 may be controlled. For example, a power for accelerating the ions may be applied to the plurality of plates 600 of the first focusing unit 61, and a power for focusing the ion beams may be applied to the plurality of plates 600 of the second focusing unit 62. A detailed description of the powers for accelerating or focusing the ions will be omitted since it is well known to those skilled in the art.

In an embodiment, the high throughput apparatus for multiple sample analysis may further comprise an ion trap for trapping the ions produced from the sample. The ion trap may be a device for trapping charged particles such as ions in the ion trap using an electromagnetic field. For example, the ion trap may be a Paul trap or other adequate devices.

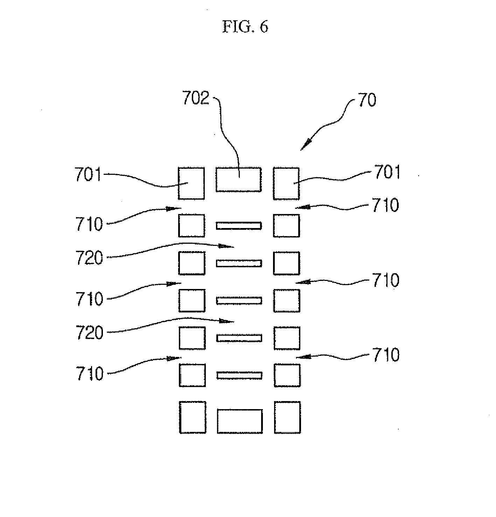

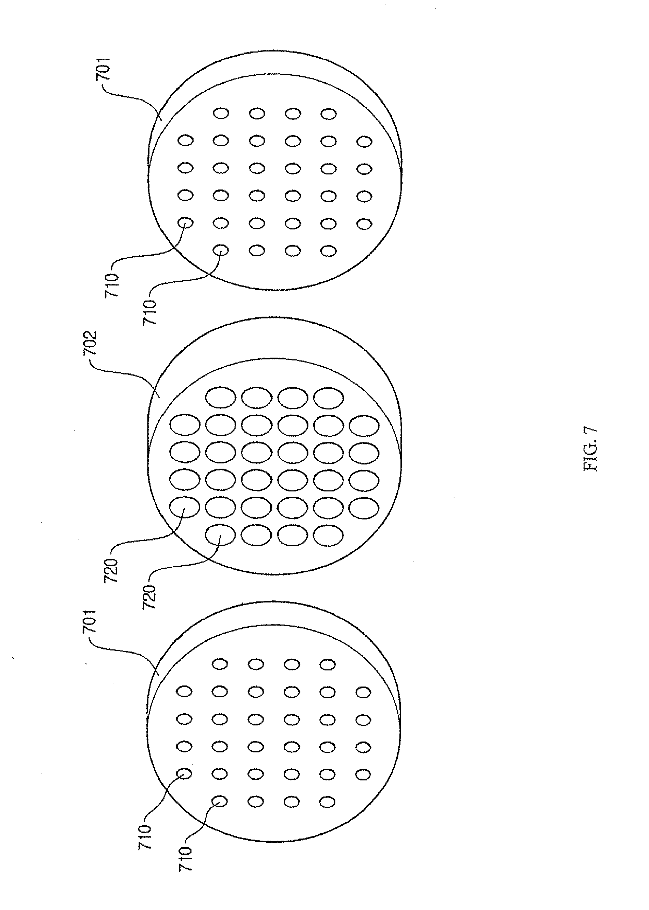

FIG. 6 is a cross-sectional view of an ion trap 70 included in a high throughput apparatus for multiple sample analysis according to an embodiment, FIG. 7 is a schematic exploded perspective view of the ion trap 70 illustrated in FIG. 6. FIG. 6 shows a cross-sectional view of the ion trap 70 along a direction perpendicular to the path of the laser beams and ions.

Referring to FIGS. 6 and 7, the ion trap 70 may comprise a plurality of first plates 701 spaced apart from each other and arranged to face each other along a direction and a second plate 702 provided between the plurality of first plates 701. The first plate 701 and the second plate 702 may be made of metal or other suitable conducting material. The diameter and thickness of the first and second plates 701, 702, the spacing between the first and second plates 701, 702, or the like may be determined adequately based on the characteristics of the ions to be trapped.

A plurality of first holes 710 arranged in an array pattern may be formed on each of the first plates 701. And, a plurality of second holes 720 arranged in an array pattern may be formed on the second plate 702. The array patterns of the plurality of first holes 710 and the plurality of second holes 720 may be substantially the same. Further, the array patterns of the plurality of first holes 710 and the plurality of second holes 720 may correspond to the arrangement of the plurality of lens of the lens array and/or the arrangement of the plurality of holes of the focusing unit.

By controlling an electromagnetic field applied to the plurality of first plates 701 and the second plate 702 of the ion trap 70, the ions produced from the sample may be trapped in the ion trap 70.

In an embodiment, to create a trapping field for trapping the ions, the second hole 720 of the second plate 702 may have a diameter larger than that of the first hole 710 of each of the first plates 701 enclosing the second plate 702. When the same voltage is applied to the first plates 701 and the second plate 702, the electric field formed in the second hole 720 becomes relatively weaker than that formed in the first hole 710. As a result, the ions may be trapped in the area of the second hole 720.

The above-described configurations of the trapping field and the ion trap 70 to generate the trapping field are only exemplary. The configuration of the ion trap 70 is not limited to the configuration disclosed herein and may be configured otherwise to appropriately trap ions.

The trapped ions may be selectively emitted from the ion trap 70. For example, an RF signal may be applied to the first plates 701 and the second plate 702 from an external power source (not shown). In this case, the ion trap 70 may be configured such that only the ions with specific mass corresponding to the applied RF signal are selectively emitted through the second holes 720 of the second plate 702.

Using the high throughput apparatus for multiple sample analysis according to the described embodiments, a sample with a large area or a large number of samples may be ionized at the same time since focused laser beams may be applied to the sample in an array pattern.

When the high throughput apparatus for multiple sample analysis is applied for matrix-assisted laser desorption/ionization (MALDI) mass spectrometry, the speed of mass analysis may be improved because a sample with a large area or a large number of samples may be ionized at the same time. For example, by detecting the ions produced from the sample by the high throughput apparatus for multiple sample analysis using a detector such as a microchannel plate (MCP), mass distribution of the sample may be analyzed.

Further, the high throughput apparatus for multiple sample analysis may be applicable to time-of-flight (TOF) mass spectrometry whereby the focused ion beams are propagated for a predetermined distance and the mass of the ions is analyzed based on the propagation time. In addition, if the ion trap is used, only the ions with specific mass may be selectively emitted from the ion trap for analysis.

Besides, the high throughput apparatus for multiple sample analysis may be applicable to MALDI imaging whereby an image corresponding to the mass distribution of the sample is obtained by irradiating laser to the sample while moving the sample in a controlled manner.

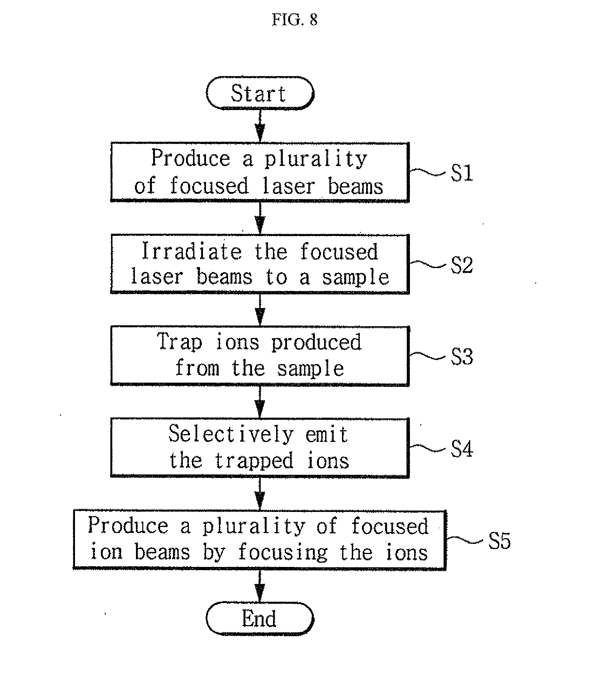

FIG. 8 is a flow chart of a high throughput method for multiple sample analysis according to an embodiment. For convenience of explanation, the high throughput method for multiple sample analysis according to this embodiment will be described referring to FIGS. 1 and 8.

Referring to FIGS. 1 and 8, laser 11 irradiated from a laser light source 10 may be focused to produce a plurality of focused laser beams 12 (S1). The plurality of focused laser beams 12 may be arranged on a plane in an array pattern.

Then, the plurality of focused laser beams 12 may be irradiated to a sample 2 (S2). The sample 2 may include a matrix for facilitating the ionization by laser. When the plurality of focused laser beams 12 are irradiated to the sample 2, the sample 2 may be ionized at the region where the laser beams 12 are irradiated and ions may be produced as a result thereof.

In an embodiment, the ions produced from the sample 2 may be trapped in an ion trap 70 (S3). Since the ions produced from the sample 2 have high temperatures, the ions may be cooled by trapping the ions in the ion trap 70.

Then, the ions trapped in the ion trap 70 may be selectively emitted (S4). For example, among the ions trapped in the ion trap 70, only the ions with specific mass may selectively emitted. However, the process S4 may be omitted if the selective emission of ions is unnecessary. Further, the process S3 may also be omitted so that subsequent processes may be performed without trapping the ions.

Then, the ions may be focused to produce a plurality of ion beams (S5). The plurality of ion beams may be arranged on a plane in an array pattern. For example, the array pattern of the plurality of ion beams may be the same as that of the plurality of focused ion beams irradiated to the sample.

While the exemplary embodiments have been shown and described, it will be understood by those skilled in the art that various changes in form and details may be made thereto without departing from the spirit and scope of the present disclosure as defined by the appended claims.

In addition, many modifications can be made to adapt a particular situation or material to the teachings of the present disclosure without departing from the essential scope thereof. Therefore, it is intended that the present disclosure not be limited to the particular exemplary embodiments disclosed as the best mode contemplated for carrying out the present disclosure, but that the present disclosure will include all embodiments falling within the scope of the appended claims.

## Claims

1. A high throughput apparatus for multiple sample analysis comprising:
a laser light source;
a lens array configured to focus laser irradiated by the laser light source into a plurality of focused laser beams; and
a focusing unit disposed between the lens array and a sample, the focusing unit configured to focus ions produced from the sample by the plurality of focused laser beams into a plurality of ion beams.

2. The high throughput apparatus for multiple sample analysis according to claim 1, wherein the lens array comprises a plurality of lenses arranged in an array pattern, and wherein each of the plurality of lenses focuses the laser irradiated by the laser light source into the focused laser beam.

3. The high throughput apparatus for multiple sample analysis according to claim 1, wherein the focusing unit comprises:
a plurality of plates spaced apart from each other and arranged to face each other along a direction; and
a plurality of holes formed on each of the plurality of plates and arranged in an array pattern.

4. The high throughput apparatus for multiple sample analysis according to claim 3,
wherein the focusing unit focuses the ions produced from the sample into the plurality of ion beams using a power applied to the plurality of plates, and
wherein each of the plurality of ion beams passes through each of the plurality of holes.

5. The high throughput apparatus for multiple sample analysis according to claim 4, wherein the plurality of plates comprise:
a plurality of first plates to which a power for accelerating the ions produced from the sample is applied; and
a plurality of second plates to which a power for focusing ions that pass through the plurality of first plates into the plurality of ion beams is applied.

6. The high throughput apparatus for multiple sample analysis according to claim 1, which further comprises an ion trap disposed between the focusing unit and the sample and configured to trap the ions produced from the sample.

7. The high throughput apparatus for multiple sample analysis according to claim 6, wherein the ion trap comprises:
a plurality of first plates spaced apart from each other and arranged to face each other along a direction;
a plurality of first holes formed on each of the plurality of first plates and arranged in an array pattern;
a second plate disposed between the plurality of first plates; and
a plurality of second holes formed on the second plate and arranged in an array pattern.

8. The high throughput apparatus for multiple sample analysis according to claim 7, wherein the ion trap traps the ions produced from the sample using an electromagnetic field applied to the plurality of first plates and the second plate, and
wherein each of the plurality of second holes has a diameter larger than that of each of the plurality of first holes.

9. The high throughput apparatus for multiple sample analysis according to claim 6, wherein the ion trap selectively emits the trapped ions according to a power applied to the ion trap.

10. A high throughput method for multiple sample analysis, comprising:
producing a plurality of focused laser beams by focusing laser;
ionizing a sample by irradiating the plurality of focused laser beams to the sample; and
producing a plurality of ion beams by focusing ions, wherein the ions are produced from the sample by the plurality of focused laser beams.

11. The high throughput method for multiple sample analysis according to claim 10, wherein the plurality of focused laser beams and the plurality of ion beams are respectively arranged in an array pattern.

12. The high throughput method for multiple sample analysis according to claim 10, which further comprises, after the ionizing the sample, trapping ions produced from the sample using an ion trap.

13. The high throughput method for multiple sample analysis according to claim 12, which further comprises, after the trapping the ions using the ion trap, selectively emitting the trapped ions from the ion trap.
